# EUROPEAN PATENT APPLICATION

(11) **EP 0 823 632 A1**
(43) Date of publication of application: **11.02.1998**
(21) Application number: 95941728.8
(22) Date of filing: 26.12.1995
(51) Int. Cl.: G01N 33/00, G08B 17/117

(54) **DIGITAL ATMOSPHERIC ANALYSER WITH LIMITED INMISSION LEVEL AND REGULATION OF THE EMISSION LEVEL**

(71) Applicant: Ingenieria de los Gases en el Medio Ambiente, S.L., 28805 Alcala de Henares (ES)
(72) Inventor: GALINDO FERNANDEZ, Jose Antonio, E-28805 Alcala de Henares (ES)
(74) Representative: Perez Bonal, Bernardo
(86) International application number: ES9500152
(87) International publication number: WO9724614

(57) **Abstract**

The analyzer is intended to analyse, quantify with digital identification and transmit automatically and autonomously, in a continuos mode and in real time, the levels of spill emissions, compared in moisture and temperature, inside a chamber designed appropiately for a forced flow, by alternative aplication of a thermal differential or by using impulse or extraction mechanical means, with a micrpprocessor having graphic recording means, remote service means, acoustic, light and graphic information means, for information transmission, and which is comprised of electronic and solid state components which are integrated and encapsulated and chained for continued security.

## Description

### OBJECT OF THE INVENTION

The invention proposed consists in a digital atmosphere analyzer with limited inmission level and adjustable emission level designed to analyze, quantify, digitally identify, and transmit in an automatic and autonomous manner, continuously and in real time, the emission levels in the ambient air in a given enclosure by means of a permanent interactive system which not only provides the orders for controlling the agent causing the risk but also warns visually, and optionally acoustically, and allows the various levels to be displayed in the digital counter, providing an appropriate recording.

It incorporates conventional recording, remote service and acoustic data means, both luminous and graphic, data conveyance means (magnetic and other), external connection jack for individually inspecting all the components, thereby enabling the introduction of redundant security means incorporated to an electronic and transistorized microprocessor with encapsulated and chained integrated components arranged in series to guarantee adequate operation, fitted with anti-explosion protection and powered through rectified direct 12 V current via a power unit, optionally fitted with an additional 12 V safety unit consecutively filtered by three-scale rupture, maintaining 12 V for manoeuvre services, 9 V for the safety barrier and 5 V for measurement operations, said microprocessor incorporating a digital counter which may optionally be remotely located and may even be portable, said inmission and emission analysis being implemented by comparing humidity and temperature values inside a chamber of an appropriate design for a forced flow produced by a thermal differential or alternatively by an extractor or some other powered driving means.

### BACKGROUND OF THE INVENTION

A multiplicity of gas detectors is available, practically all of such gases having carbon and hydrogen components involved in the creation of contaminating and explosive atmospheres, etc., the detectors incorporating ionic and electrochemical sensors or reference electrodes such as, for instance, those built on the basis of a tin oxide semiconductor with level preselection (US 5218347), said semiconductor and a catalytic electrode (EP 492700), an electrochemical type selenium sensor (US 4007096 and US 3909384) or a sensor within the anfigenous family which detects variations in electric conductivity under the effect of reducer or oxidizing gases (ES 535575).

In certain cases, these sensors are integrated in gas content analyzers having these characteristics, some of them even being capable of performing the analysis in a continuous manner.

Such is the case of Spanish invention patent No. 518.301, filed by the same inventor, called "Environment analysis apparatus with signalling device in the event of atmosphere composition variations within a given enclosure". This invention provides, among other data, the sensing of all kinds of gases through the functional combination of two sensors independent of the percentage variations in environment oxygen, one for detecting the displacement of the oxygen originated by the presence of combustible of explosive gases and the other for toxic gases, plus a control and alarm signal generating electronic circuit, plus a signal emitter.

By the same inventor, Spanish patent application No. 9501503, "Permanent interactive analytic station for controlling hydrocarbon gas and vapor environment emission levels, with preset inmission levels", incorporates, with the sensors, two independent pneumatic circuits, one for diluting said vapors by controlled airing and the other for making them inert, a pneumatic station being applied to the inspection well for the receptacles housing said fuel tanks.

Patents EP 342513, EP 327089 and US 4827154 also provide ventilation system activation means for gas sensors.

Furthermore, the patent for analyzer SU911298, apart from the components already described, incorporates a proportional pressure regulating means which controls the flow of gas as a function of the pressure through a pass valve connected to a tank and fitted with an intermediate heater for the flow which prevents the gas from freezing during the transit period.

In summary, known detectors not only fail to limit the inmission level but provide only a presence indication by a Yes or a No, without referencing the contaminating product to a specific gas.

The applicant is unaware of the existence of registers, applications or products in the market relating to atmosphere digital analyzers which analyze, quantify and transmit emission data in an automatic and autonomous manner, continuously and in real time, providing a limited inmission level and an adjustable emission level through a permanent interactive system.

### DESCRIPTION OF THE INVENTION

The invention that is the object of the present specification relates to a digital atmosphere analyzer with a limited inmission level and adjustable emission level designed to detect contaminating product emissions to the exterior or within closed environments, very particularly comprising carbohydrogenated compounds (energetic gases, ethers, alcohols and others), although other compounds are not excluded, by means of sensors applicable to the various gas families, characterized, as previously stated, in that the analyzer is of a digital construction designed to permanently identify potential risk levels and to continuously and in real time assess and convey inmission and emission level data concerning the gases dumped to said environment via technology capable of providing accurate information and solutions according to each situation.

It is fitted with a permanent interactive system which, in addition to enabling the orders needed for controlling the risk causing agent, provides a visual warning, and optionally an acoustic warning, and, when requirements so dictate, records the various risk levels for subsequent analysis.

The system provides a permanent assessment of both the emission level, namely the concentration and/or the mass in a contaminant dumped to the atmosphere during a given period or time or according to a production unit, and the inmission level, namely the quantity of said existing contaminant per air volume, conveying said inmission and emission data in real time.

The system is equipped with a comparative analysis chamber for evaluating humidity and temperature data via a sequential and permanent thermistor, said chamber having its supposedly contaminated air entry and exit holes facing each other, their respective diameters being substantially different, the chamber being designed so that, upon incorporating an electric resistor designed to increase the chambers's ambient temperature in 20°C, a forced flow is generated which, alternatively to the heating resistor, may be originated through appropriate external equipment such as a mini-extractor or other powered driving means.

Analysis within the chamber is conducted by catalysis, presetting a basic air reference value equal to the unit in regard to oxygen and nitrogen, so that any variation introduced by the interfering agent is either displayed or not by a digital counter, the evaluated gas mixture being conveyed back to the exterior.

A microprocessor is provided for the programmed adjustable measurement of the reference value in regard to the analyzed value, so that the counter incorporated in the system causes the maximum value in its digital scale to coincide with the lower explosive limit (LEL) of the gas being analyzed - e.g. in the event of butane being present in the air, the maximum value would be 1.82% of butane in the air.

In the case of methane being present in the air, methane being a standard reference gas in all test labs, said maximum inmission value as stated in the previous case would correspond to 5% methane.

The emission level is established as a percentage of the maximum value or limit, coinciding with the start of the toxicity risk and currently proposed as 25% of said maximum value either displayed or not by the digital counter, as in the previous case, said value enabling the order to discontinue the supply of power or, as necessary, to apply corrective action for closing all accesses and activating ambient air extractors, visual and acoustic warnings, etc.

Thus, homogenization of gas in air need not be implemented within the enclosure, as is the normal case in all other sensing procedures, the air being forced to pass continually through the analyzer and its proportions being permanently known values without relying on the typical Yes or No response from the conventional detector, emission fluctuations being furthermore displayed when they are well below the action limits, thus generating a psychological sense of confidence in the user as a result of permanent level indications being displayed in the digital counter.

The invention is enabled and completed by conventional graphic recording means, an adequate program, remote service means, acoustic, visual and graphic information, and data transmission means (magnetic and other).

Depending on the application foreseen, whether industrial or domestic, two embodiment variations are possible, the first separating the chamber from the microprocessor and optionally fitted with a common microprocessor - sequentially acting on a multiplicity of chambers - and sensors, or a combination thereof.

The domestic variation comprises a microprocessor, a counter and a chamber forming a single unit.

Concerning the action variations, the unit orders either the closure of the feeding valve assembly or the gas cylinder regulator, in the case of gas being supplied for domestic use.

### DESCRIPTION OF THE DRAWINGS

In order to complement the description being provided and facilitate a better and fuller comprehension of the characteristics of the invention, a set of drawings is attached to this specification as an integral part thereof, wherein the following is represented in an illustrative an non-limiting character:

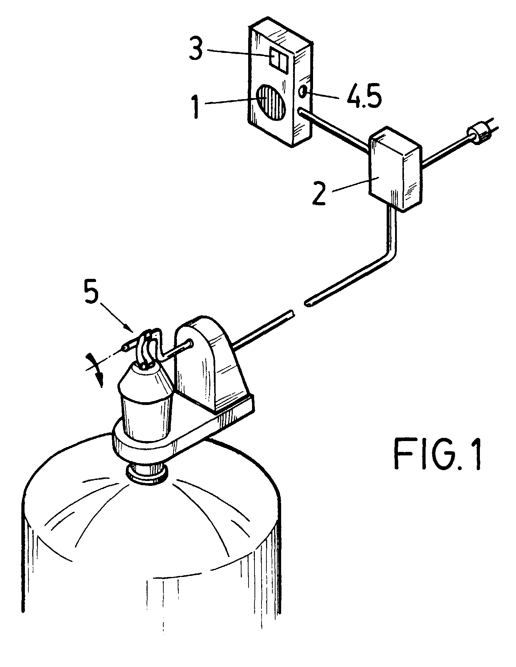

Figure 1 is a perspective schematic diagram of the operation and the components in a basic equipment of the invention, comprising a compact analyzer integrating a microprocessor and analysis sensor, and an external power unit, further showing an example of the gas cylinder regulator closure means in the form of a power-driven lever.

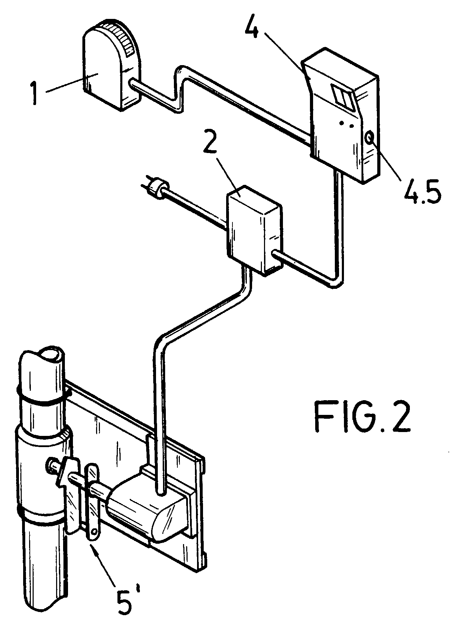

Figure 2 shows similar closure means for an industrial facility, fitted with a split equipment, the microprocessor power unit and the analyzing sensor being separated.

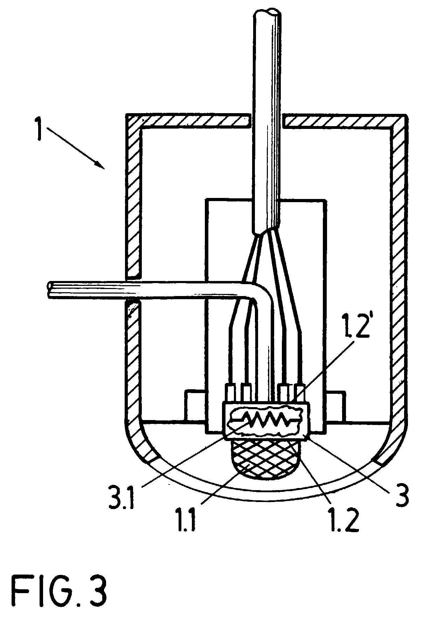

Figure 3 is a detail view of the gas analyzing chamber for domestic or industrial use, comprising a conventional sensor, an air admission filter with forced extraction duct, an electric power supply facility and a measurement outlet.
Figure 4 shows a construction diagram of a possible embodiment of a digital counter which in this case incorporates, in addition to the double consecutive operation graphic displays, the known door barriers and transistors and the means of regulating the inmission limit scale through a variable resistor.

### PREFERRED EMBODIMENT OF THE INVENTION

In the light of the above figures, it can be seen that the invention being advocated consists in a digital atmosphere analyzer with limited inmission level and adjustable emission level designed to detect environment contaminants in enclosures, fitted with sensors applicable to each gas family being analyzed, characterized in that the analyzer, of a digital construction, permanently identifies potential risk levels and is designed to assess and transmit, continuously and in real time, the inmission and emission levels of the gases dumped to said environment air by means of a permanent interactive, electronic and transistorized system comprising integrated encapsulated and chained components for continuous safety assessment, arranged in series to guarantee reliable operation, fitted with anti-explosion protection and powered through rectified direct 12 V current, and an optional 12 V safety unit consecutively filtered by three-scale rupture, maintaining 12 V for manoeuvre services, 9 V for the safety barrier and 5 V for measurement operations, the system comprising a chamber (1) and a microprocessor (2) to enable, in real time, the orders needed for controlling the risk causing agent and furthermore provides a visual warning, and optionally an acoustic warning, and, when retirements so dictate, records the various risk levels for subsequent analysis.

The chamber (1) provides a comparative humidity analysis between 5 and 95% (as a safety limit) and a temperature analysis between 0 and 40°C (false measurement limit caused by expansion of the gas molecules), and is fitted with a contaminated air admission filter (1.1), entry (1.2) and exit (1.2') holes with respective 10 and 3 mm diameters (as in this preferred example, said same diameter quotient being maintained, if possible, in other cases), conducting the analysis by means of a sequentially permanent thermistor and incorporating a conventional sensor (3), an electric resistor (3.1) designed to increase the internal temperature in 20°C, or else replacing the resistor by a mini-extractor or some other powered impeller means which generates a forced flow on which the analysis by catalysis is implemented, presetting a basic air reference value equal to the unit in regard to the oxygen, the nitrogen or the replacement controlled atmosphere, so that any variation introduced by the interfering agent is either displayed or not by the digital counter (4), the evaluated gas mixture being then conveyed back to the exterior.

The microprocessor (2) is adjustably programmed for measuring the gas reference value in respect to the analyzed value, so that the counter (4) incorporated in the system causes the maximum value in its digital scale to coincide with the lower explosive limit (LEL) of the gas being analyzed through ionic conductivity, whereas a percentage of the maximum value or limit is established for the fixed emission level, coinciding with the start of the toxicity risk proposed at 25% of said maximum value, said value enabling the orders to discontinue the supply of power or to close the feed valve (5) or the gas cylinder regulator ducts (5'), etc., as necessary, as well as applying corrective action, closing accesses and activating the ambient air extractors and the visual and acoustic warnings, etc.

The digital counter (4) compares the analogic ionic reading and converts it to digital with the use of two consecutive functions (4.1) and (4.1'), the conventional door barriers (4.2), the transistors, the resistors, the light indicators (4.3) with at least two digits and a variable resistor (4.4) which regulates the inmission limit scale.

In addition to said sensor, appropriate for each gas family, the system incorporates a conventional graphic recording means, an adequate program, a remote service means, acoustic, visual and graphic information, a data transmission means (magnetic and other), an external jack (4.5) or connection, and individual checking means for all the components, thereby enabling the introduction of redundant safety means for special very high risk applications requiring said redundant safety according to regulations.

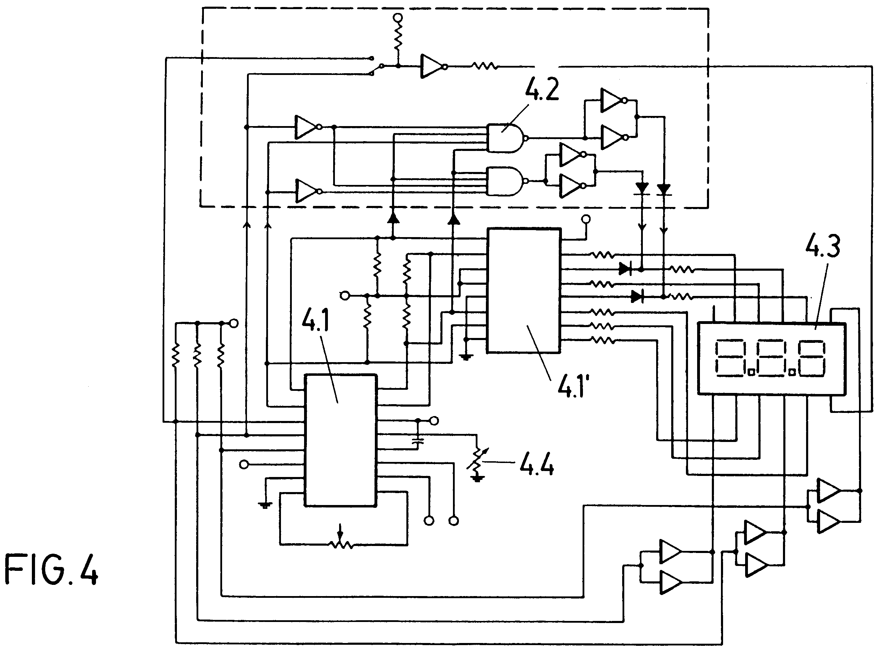

The example diagram in figure 4 may be modified in its electronic circuit when the digital representation is implemented over three displays with the represented scale variation, i.e. when the reading shown is millesimal, the circuit shown will house the total number of components and, when the reading display is a unit, the modification of the circuit will eliminate all the derived components to a digit, allowing two of them for service and the values of the associated components for the scale to be recorded.

Version 1 - Domestic, integrating the microprocessor, the counter and the chamber in a single element in the embodiment described for the preferred example.

Version 2 - Industrial, the chamber being arranged independent from the microprocessor, which may optionally be arranged in common and sequentially for a multiplicity of chambers which may even be installed in different buildings, or simple sensors or combinations of both, incorporating means auxiliary to the ones described, such as inertizing means, among others.

In very polluted industrial environments, the contaminants are separated prior to the gases being introduced in the chamber in order to avoid the analyses from being deformed or impaired, using conventional prior filtration, not shown in the figures, utilizing a sintering material and implementing particulate retention through molecular impact.

Version 3 - A further embodiment, for either industrial or domestic applications, not reflected because of its descriptive simplicity, would involve eliminating the digital counter (4) in a version that would only include the Yes/No responses for action orders based on inmission values at each moment, the digital counter (4) being used only occasionally for checking and manually controlling the analyzer through the jack (4.5).

This description is not made more extensive in the understanding that an expert on the subject will have sufficient information to perceive the scope of the invention and the advantages deriving therefrom, and also to reproduce said invention.

It should be understood that, provided the essential nature of the invention is not altered, the materials, shape, size and arrangement of the elements may vary.

The descriptive terms and their meaning must always be construed in a non-limiting sense.

## Claims

1. Digital atmosphere analyzer with limited inmission level and adjustable emission level for detecting environment contaminants in enclosures, being fitted with sensors applicable to each gas family being analyzed and designed to permanently identify, not only within enclosures but also in the free atmosphere, potential risk levels, and to assess and transmit, continuously and in real time, the inmission and emission levels of the emissions dumped to said environment air by means of a permanent interactive system which enables not only the orders needed for controlling the risk causing agent but also provides a visual warning and optionally an acoustic warning, and, when requirements so dictate, records the various risk levels for storage and subsequent evolution analysis, in addition to implementing corrective actions or the closing of accesses, essentially characterized in that it forms a compact unit comprising:
- a chamber (1) for analyzing the interfering gas propelled inside the chamber by self-convection through a temperature differential or by means of a vacuum mini-pump or a mini-extractor, incorporating an electric resistor (3.1) that increases the internal temperature in a preset value and establishes predefined humidity conditions for analyzing the variation of the oxygen and nitrogen percentage in respect to the basic reference value equal to the unit corresponding to 21% oxygen and 78% nitrogen,
- a digital counter (4) operating in real time which displays the reference variations in oxygen and nitrogen concentration caused by the interfering agent,
- a microprocessor (2), adjustably programmed for measuring the gas reference value in respect to the value of the gas being analyzed, for the emission level, causing the maximum value in the counter (4) scale to coincide with the lower explosive limit (LEL) of the gas being analyzed, whereas a percentage of the maximum value or limit is established for the emission level, coinciding with the start of the toxicity risk, stated at 5 to 95% of said maximum value,
- an external jack (4.5) or connection for individually checking all the components, thereby enabling the introduction of redundant safety means.

2. Digital atmosphere analyzer with limited inmission level and adjustable emission level, according to claim 1, characterized in that the electronic and transistorized microprocessor (2) is fitted with encapsulated integrated components arranged in series for anti-explosion protection, the microprocessor (2) being powered through rectified direct current and fitted with an optional safety unit operating at the same voltage, consecutively filtered by three-scale rupture and maintaining the higher voltage for manoeuvre services, the intermediate voltage for the safety barrier and the lower voltage for measurement operations, all of which are arranged in a continuous series sequence.

3. Digital atmosphere analyzer with limited inmission level and adjustable emission level, according to the first claim, characterized in that the digital counter (4) compares the analogic ionic reading and converts it to digital when the digital data is shown on three displays with the variation of the adequate scale, i.e. when the reading shown is millesimal, in addition to two consecutive functions (4.1) and (4.1') and the conventional door barriers (4.2), the transistors, the resistors, the three-digit luminous indicators (4.3) and a variable resistor (4.4) which regulates the inmission limit scale, subject to a continuous series sequence.

4. Digital atmosphere analyzer with limited inmission level and adjustable emission level, according to the foregoing claim, characterized in that, when the reading display is based on units, the modification of the circuit will eliminate all the derived components to a digit, allowing two of them for service and the values of the associated components for the scale to be recorded.
